# EUROPEAN PATENT APPLICATION

(11) **EP 4 357 329 A1**
(43) Date of publication of application: **24.04.2024**
(21) Application number: 22824866.2
(22) Date of filing: 07.06.2022
(51) Int. Cl.: C07C 31/135, A23L 27/00, A23L 27/20, A61K 8/34, A61Q 13/00, C07B 61/00, C07C 29/19, C11B 9/00, C11D 3/50

(54) **ALICYCLIC ALCOHOL, ALICYCLIC ALCOHOL COMPOSITION, AND PERFUME COMPOSITION**

(30) Priority: 14.06.2021 JP 2021098808
(71) Applicant: MITSUBISHI GAS CHEMICAL COMPANY, INC., Chiyoda-ku Tokyo 100-8324 (JP)
(72) Inventor: TOMAKI, Keisuke, Hiratsuka-shi, Kanagawa 254-0016 (JP); SHIRAI, Shinyou, Kurashiki-shi, Okayama 712-8525 (JP)
(74) Representative: Hoffmann Eitle
(86) International application number: PCT/JP2022/022904
(87) International publication number: WO 2022/264874

(57) **Abstract**

An alicyclic alcohol represented by General Formula (1) below:

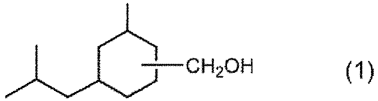

## Description

### Technical Field

The present invention relates to an alicyclic alcohol, a method for producing the alicyclic alcohol, an alicyclic alcohol composition containing the alicyclic alcohol, and a fragrance composition.

### Background Art

Some alicyclic alcohols are known to be useful as compounded fragrance ingredients. For example, Non-Patent Literature 1 describes that p-isopropylcyclohexylmethanol (Mayol) has a floral fragrance, that p-isopropylcyclohexanol has a floral fragrance obtained by adding mint to lilac, rose, and geranium, and that 2,4-dimethyl-3-cyclohexene-1-methanol has a fresh green and floral fragrance reminiscent of hyacinth or lily-of-the-valley. Non-Patent Literature 1 describes that various alicyclic alcohols including these compounds are useful as compounded fragrance ingredients, and also describes applications such as skincare products.

Patent Document 1 discloses 4-isobutylcyclohexylmethanol and a fragrance composition having fresh floral, rose-like, citronellal-like, weak lily-of-the-valley-like, and fragrant olive-like aromatic notes.

### Citation List

### Non-Patent Literature

Non-Patent Literature 1: Motoichi Indo, "Zoho Kaitei ban, Gosei Koryo: Kagaku to Shohin Chishiki (Enlarged and Revised Edition, Synthetic Fragrance: Chemistry and Product Knowledge)", The Chemical Daily Co. Ltd., 1996, pages 54 to 56

### Patent Document

Patent Document 1: JP H01-207252 A

### Summary of Invention

### Technical Problem

Alicyclic alcohols for fragrance use are used in various fields, such as cosmetic products including skin care products as described above, as well as fragrance products, detergents, sundry goods, pharmaceuticals, and food products. To increase the value of the end products, new scents are being developed, and fragrances having a novel scent are in demand.

Accordingly, an object of the present invention is to provide an alicyclic alcohol which has a floral and fruity scent, can emphasize a juicy feeling, a floral feeling, and a fruity feeling in addition to the scent in a compounded fragrance, is useful as fragrance, and is also useful as an ingredient for a compounded fragrance. Another object of the present invention is to provide a method for producing the alicyclic alcohol, an alicyclic alcohol composition containing the alicyclic alcohol, and a fragrance composition.

### Solution to Problem

The present inventors have synthesized and/or blended various alicyclic alcohols and compositions containing a plurality of those alicyclic alcohols and evaluated their aromas, and found that specific alicyclic alcohols and compositions containing those alicyclic alcohols have an excellent aromatic note and are also excellent as ingredients for compounded fragrances, and completed the present invention.

That is, the present invention includes as follows.
[1] An alicyclic alcohol represented by General Formula (1) below: 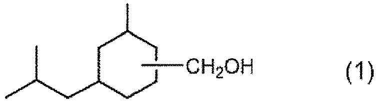
[2] The alicyclic alcohol according to [1], which is represented by General Formula (2) below: 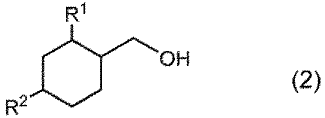 where R¹ is a methyl group and R² is an isobutyl group, or R¹ is an isobutyl group and R² is a methyl group.
[3] The alicyclic alcohol according to [1] or [2], which is represented by Formula (3) below: 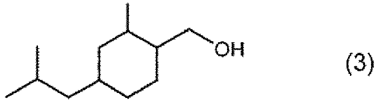
[4] The alicyclic alcohol according to [1] or [2], which is represented by Formula (4) below: 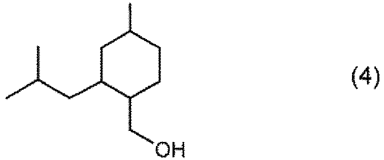
[5] An alicyclic alcohol composition containing an alicyclic alcohol represented by Formula (3) below and an alicyclic alcohol represented by Formula (4) below: 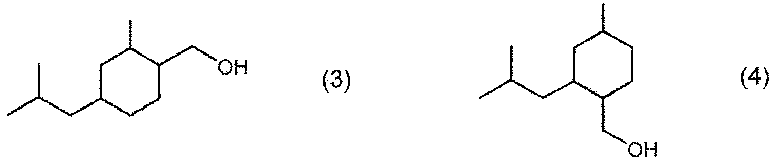
[6] The alicyclic alcohol composition according to [5], wherein [(3)/(4)], a ratio of the alicyclic alcohol represented by Formula (3) above to the alicyclic alcohol represented by Formula (4) above in terms of a peak area ratio in a gas chromatograph, is from 7/93 to 98/2.
[7] A fragrance composition containing the alicyclic alcohol described in any one of [1] to [4] or the alicyclic alcohol composition described in [5] or [6].
[8] A method for producing an alicyclic alcohol, the method including obtaining an alicyclic alcohol represented by General Formula (6) below by hydrogenating an aromatic aldehyde represented by General Formula (5) below: 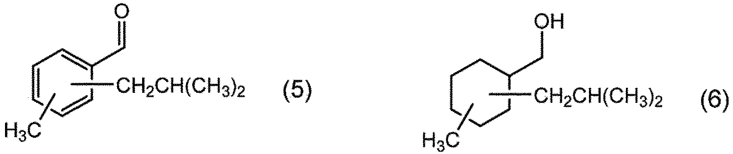
[9] The method for producing an alicyclic alcohol, according to [8], the method including obtaining an alicyclic alcohol represented by General Formula (1) below by hydrogenating an aromatic aldehyde represented by General Formula (7) below:

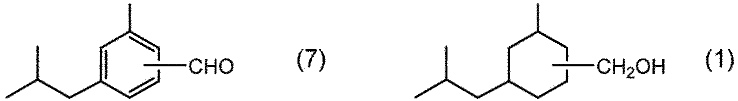

### Advantageous Effects of Invention

According to the present invention, provided is an alicyclic alcohol which is useful as fragrance since it has a floral and fruity scent, and is also useful as an ingredient for a compounded fragrance since it can emphasize a juicy feeling, a floral feeling, and a fruity feeling in addition to the scent in the compounded fragrance. Further, a method for producing the alicyclic alcohol, an alicyclic alcohol composition containing the alicyclic alcohol, and a fragrance composition are also provided.

### Brief Description of Drawings

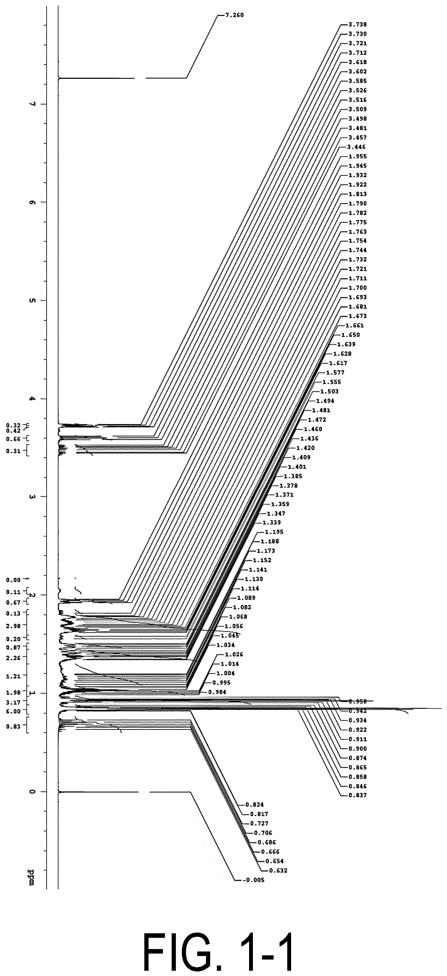

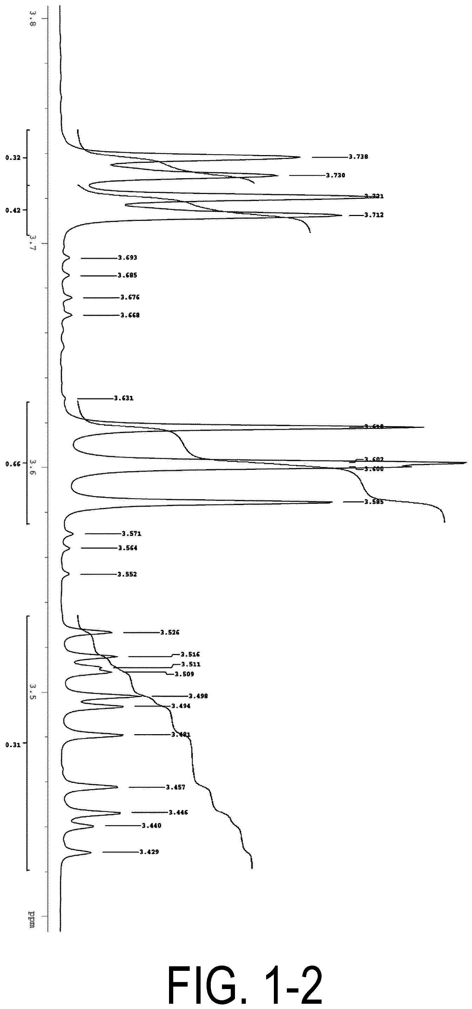

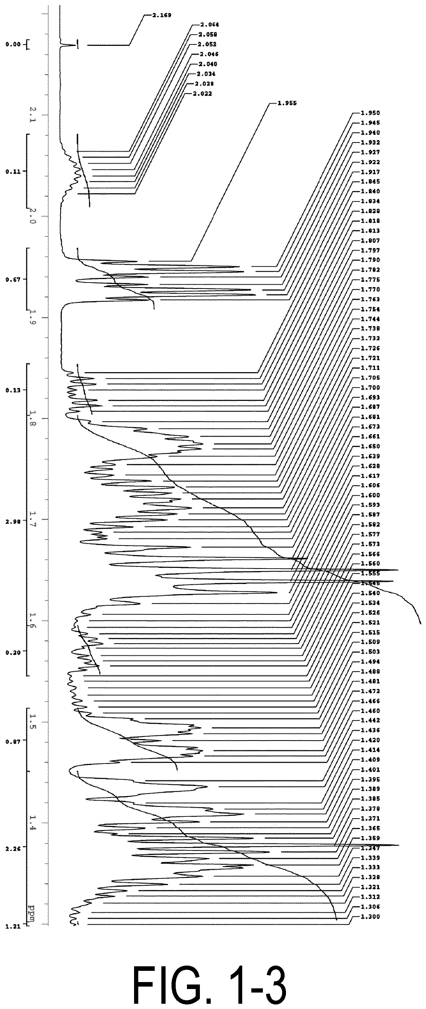

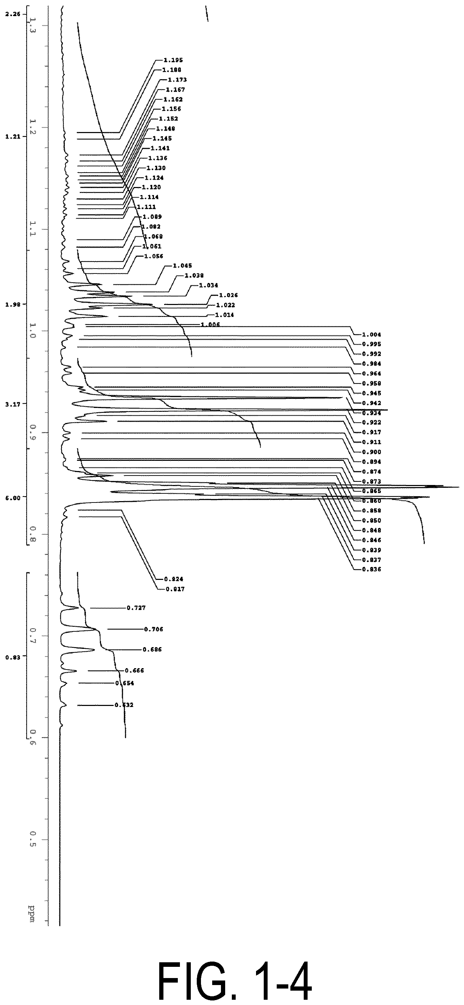

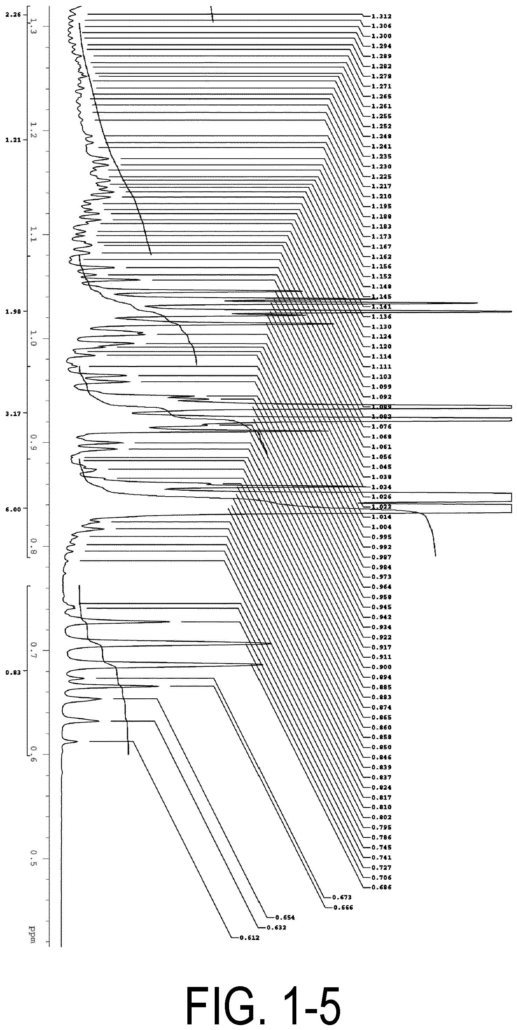

FIG. 1-1 illustrates an example of an NMR analytical result of an alicyclic alcohol (4-isobutyl-2-methylcyclohexyl)methanol (Formula (3)) of the present invention.
FIG. 1-2 is an enlarged view of a part of the NMR analytical result illustrated in FIG. 1-1.
FIG. 1-3 is an enlarged view of a part of the NMR analytical result illustrated in FIG. 1-1.
FIG. 1-4 is an enlarged view of a part of the NMR analytical result illustrated in FIG. 1-1.
FIG. 1-5 is an enlarged view of a part of the NMR analytical result illustrated in FIG. 1-1.
FIG. 2 illustrates a GC-MS analytical result of the alicyclic alcohol (4-isobutyl-2-methylcyclohexyl)methanol (Formula (3)) of the present invention.
FIG. 3-1 illustrates an example of an NMR analytical result of an alicyclic alcohol (2-isobutyl-4-methylcyclohexyl)methanol (Formula (4)) of the present invention.
FIG. 3-2 is an enlarged view of a part of the NMR analytical result illustrated in FIG. 3-1.
FIG. 3-3 is an enlarged view of a part of the NMR analytical result illustrated in FIG. 3-1.

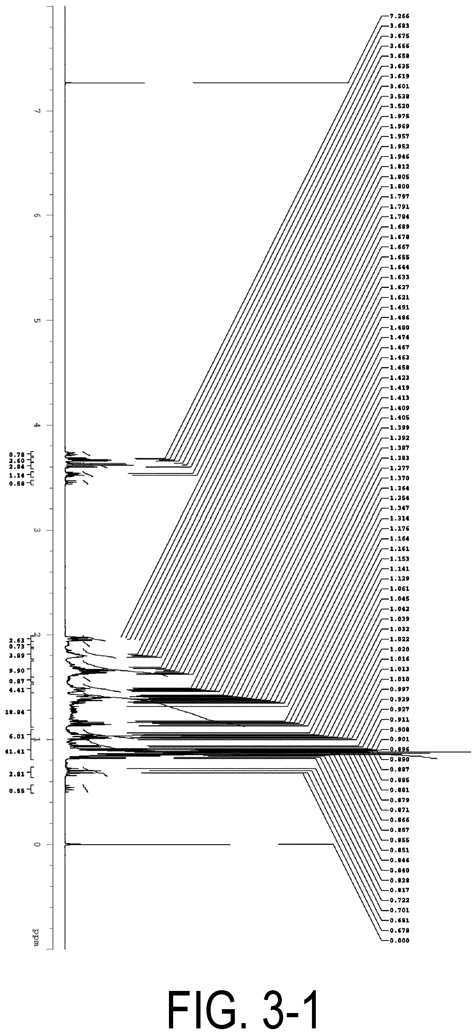

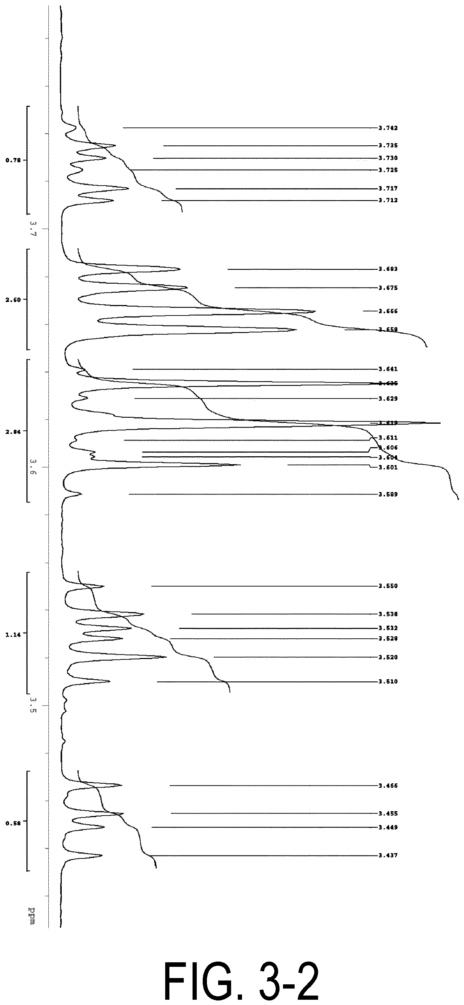

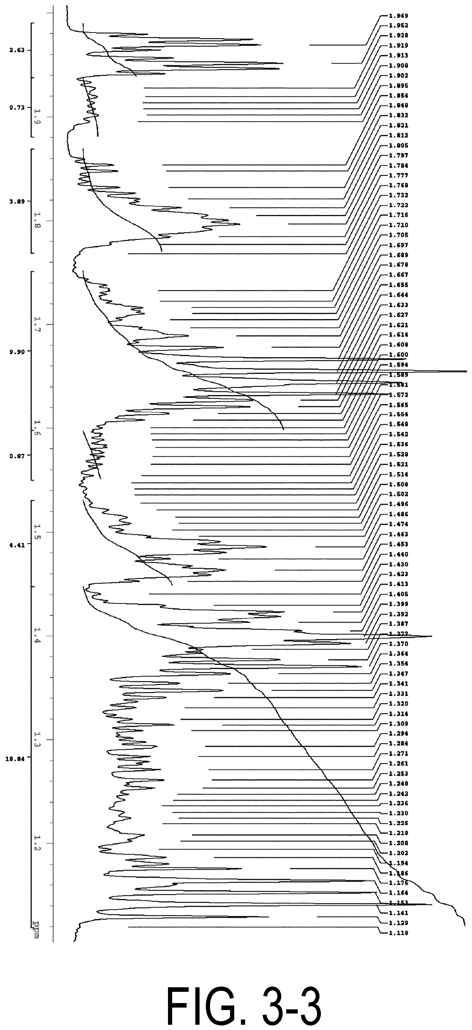

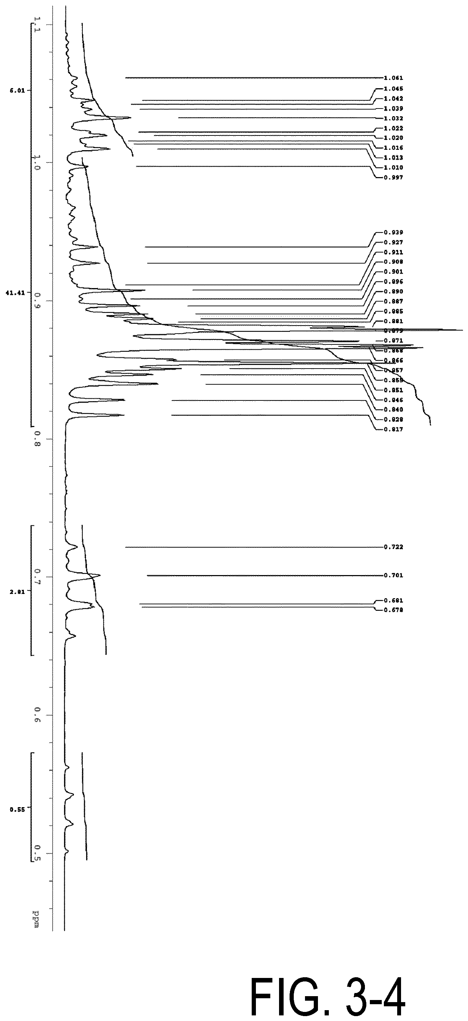

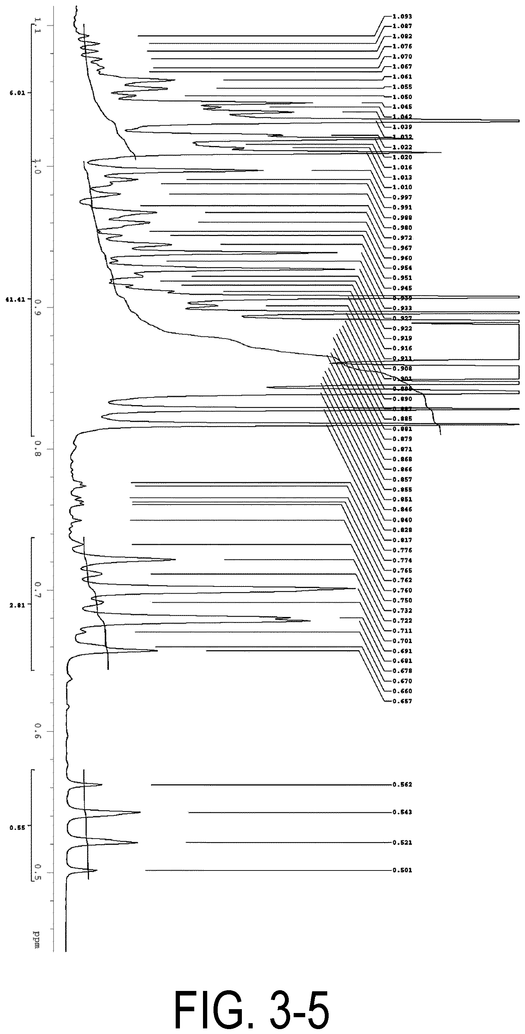

FIG. 3-4 is an enlarged view of a part of the NMR analytical result illustrated in FIG. 3-1.
FIG. 3-5 is an enlarged view of a part of the NMR analytical result illustrated in FIG. 3-1.

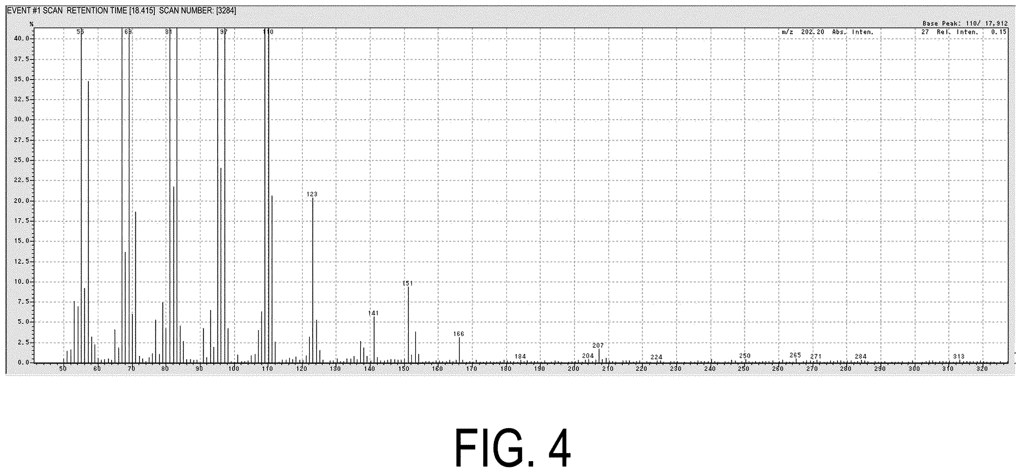

FIG. 4 illustrates a GC-MS analytical result of the alicyclic alcohol (2-isobutyl-4-methylcyclohexyl)methanol (Formula (4)) of the present invention.

### Description of Embodiments

### [Alicyclic Alcohol]

An alicyclic alcohol of the present invention is represented by General Formula (1) below:

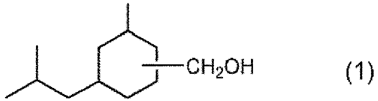

The alicyclic alcohol represented by General Formula (1) has a floral and fruity scent, and thus is useful as fragrance. The alicyclic alcohol can emphasize a juicy feeling, a floral feeling, and a fruity feeling, in addition to the above scent, and thus is also useful as an ingredient for a compounded fragrance.

The alicyclic alcohol represented by General Formula (1) is preferably obtained by a production method which will be described later.

Among the alicyclic alcohols represented by General Formula (1), an alicyclic alcohol represented by General Formula (2) below is preferable: 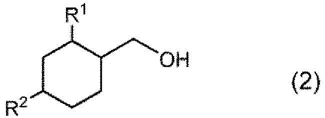 wherein R¹ is a methyl group and R² is an isobutyl group, or R¹ is an isobutyl group and R² is a methyl group.

The alicyclic alcohol represented by General Formula (1) is more preferably an alicyclic alcohol represented by General Formula (3) below or an alicyclic alcohol represented by General Formula (4) below. The alicyclic alcohol represented by General Formula (2) is an alicyclic alcohol represented by General Formula (3) below or an alicyclic alcohol represented by General Formula (4) below.

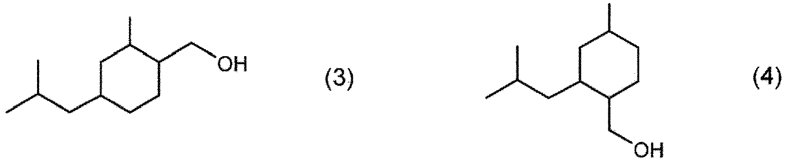

The alicyclic alcohol represented by Formula (3) has a floral and fruity scent, and thus is useful as fragrance. The alicyclic alcohol can emphasize a juicy feeling, a floral feeling, and a fruity feeling, in addition to the above scent, and thus is also useful as an ingredient for a compounded fragrance.

The alicyclic alcohol represented by Formula (4) has a floral and fruity scent, and thus is useful as fragrance. The alicyclic alcohol can emphasize a juicy feeling, a floral feeling, and a fruity feeling, in addition to the above scent, and thus is also useful as an ingredient for a compounded fragrance.

Both the alicyclic alcohol represented by Formula (3) and the alicyclic alcohol represented by Formula (4) are preferably obtained by the production method which will be described later.

### [Alicyclic Alcohol Composition]

An alicyclic alcohol composition containing two or more of the alicyclic alcohols represented by General Formula (1) above is also preferable because it exhibits the above-described effects.

Among these alicyclic alcohol compositions, an alicyclic alcohol composition containing an alicyclic alcohol represented by Formula (3) and an alicyclic alcohol represented by Formula (4):

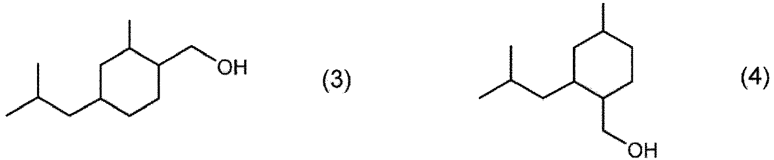

### <Composition of Alicyclic Alcohol Composition, etc.>

A total content of the alicyclic alcohol represented by Formula (3) above and the alicyclic alcohol represented by Formula (4) above in the alicyclic alcohol composition of the present invention is preferably 95% or higher, more preferably 96% or higher, and even more preferably 97% or higher in terms of a peak area proportion in a gas chromatograph. An upper limit is not limited, and may be any value as long as it is 100% or lower.

A content of the alicyclic alcohol represented by Formula (3) above in the alicyclic alcohol composition of the present invention is preferably 2% or higher, more preferably 5% or higher, even more preferably 7% or higher, still even more preferably 70% or higher, still even more preferably 80% or higher, and still even more preferably 85% or higher in terms of a peak area proportion in a gas chromatograph. Also, the content is preferably 98% or lower, more preferably 95% or lower, and even more preferably 90% or lower.

A content of the alicyclic alcohol represented by Formula (4) above in the alicyclic alcohol composition of the present invention is preferably 2% or higher, more preferably 5% or higher, and even more preferably 10% or higher in terms of a peak area proportion in a gas chromatograph. Also, the content is preferably 98% or lower, more preferably 95% or lower, even more preferably 93% or lower, still even more preferably 30% or lower, even more preferably 20% or lower, and even more preferably 15% or lower.

A ratio [(3)/(4)] of the alicyclic alcohol represented by Formula (3) above to the alicyclic alcohol represented by Formula (4) above in the alicyclic alcohol composition of the present invention is preferably from 2/98 to 98/2, more preferably from 5/95 to 98/2, even more preferably from 7/93 to 98/2, still even more preferably from 10/90 to 98/2, still even more preferably from 70/30 to 98/2, still even more preferably from 80/20 to 95/5, and still even more preferably from 85/15 to 90/10 in terms of a peak area ratio in a gas chromatograph.

The peak area proportion and the peak area ratio in the gas chromatograph are determined by a chromatograph obtained by gas chromatography.

Specifically, they are determined by a chromatograph obtained by a gas chromatography method using a nonpolar column, using FID as a detector, and measured at a temperature of from 150°C to 300°C.

More specifically, they are determined by a chromatograph measured by a method as described in the Examples.

The alicyclic alcohol composition of the present invention having the above composition has a floral and fruity scent, and thus is useful as fragrance. Furthermore, the alicyclic alcohol composition can emphasize a juicy feeling, a floral feeling, and a fruity feeling, in addition to the scent, in a compounded fragrance, and thus is useful as an ingredient for a compounded fragrance, and can be used as an aromatic component of various products.

### [Fragrance Composition]

A fragrance composition of the present invention contains the alicyclic alcohol or the alicyclic alcohol composition.

That is, a fragrance composition according to a first embodiment of the present invention contains an alicyclic alcohol represented by General Formula (1) below:

The fragrance composition according to the first embodiment of the present invention contains the alicyclic alcohol having a floral and fruity scent, and thus a juicy feeling, a floral feeling, and a fruity feeling can be emphasized in addition to the scent in a compounded fragrance (fragrance composition).

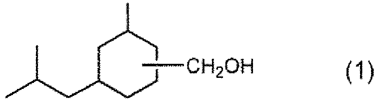

The alicyclic alcohol contained in the fragrance composition according to the first embodiment of the present invention is the alicyclic alcohol represented by General Formula (1) above, preferably the alicyclic alcohol represented by General Formula (2) above, and more preferably the alicyclic alcohol represented by formula (3) above.

The content of the alicyclic alcohol in the fragrance composition of the present invention is to be appropriately adjusted according to the type of fragrance composition, the type of target aroma, the intensity of the aroma, and the like and is preferably from 0.01 to 90 mass% and more preferably from 0.1 to 50 mass%.

A fragrance composition according to a second embodiment of the present invention contains an alicyclic alcohol composition containing two or more of the alicyclic alcohols represented by General Formula (1) above.

The alicyclic alcohol composition contained in the fragrance composition according to the second embodiment of the present invention is preferably an alicyclic alcohol composition containing an alicyclic alcohol represented by formula (3) below or an alicyclic alcohol represented by formula (4) below, and more preferably an alicyclic alcohol composition containing an alicyclic alcohol represented by formula (3) below and an alicyclic alcohol represented by formula (4) below.

The fragrance composition according to the second embodiment of the present invention contains the alicyclic alcohol composition having a floral and fruity scent, and thus a juicy feeling, a floral feeling, and a fruity feeling can be emphasized in addition to the scent in a compounded fragrance (fragrance composition).

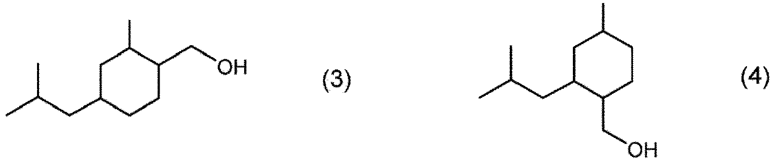

The content of the alicyclic alcohol composition in the fragrance composition of the present invention is to be appropriately adjusted according to the type of fragrance composition, the type of target aroma, the intensity of the aroma, and the like and is preferably from 0.01 to 90 mass% and more preferably from 0.1 to 50 mass%.

In the following description, the "fragrance composition of the present invention" means both the "fragrance composition according to the first embodiment of the present invention" and the "fragrance composition according to the second embodiment of the present invention".

In the fragrance composition of the present invention, the component that can be used in combination with the alicyclic alcohol or the alicyclic alcohol composition is not limited, and the following components can be indicated as examples.

For example, a surfactant, a solvent, and a fragrance substance other than the alicyclic alcohol represented by General Formula (1) above are indicated.

In particular, the component is combined with a fragrance substance other than the alicyclic alcohol represented by General Formula (1) above, and thus a floral and fruity scent can be imparted to the fragrance composition, and a juicy feeling, a floral feeling, and a fruity feeling can be emphasized. Therefore, the fragrance composition of the present invention preferably contains a fragrance substance other than the alicyclic alcohol represented by General Formula (1) above.

Examples of the surfactant include polyoxyethylene lauryl sulfate ether.

Examples of the solvent include dipropylene glycol, diethyl phthalate, ethylene glycol, propylene glycol, methyl myristate, triethyl citrate, and ethanol.

The fragrance substance other than the alicyclic alcohol represented by General Formula (1) above, which can be used in the fragrance composition of the present invention is preferably at least one selected from the group consisting of hydrocarbons, alcohols, phenols, esters, aldehydes, ketones, acetals, ketals, ethers, nitriles, lactones, natural essential oils, and natural extracts.

Specific examples of the fragrance substance other than the alicyclic alcohol represented by General Formula (1) above include hydrocarbons such as limonene, α-pinene, β-pinene, terpinene, cedrene, longifolene, and valencene; alcohols such as linalool, citronellol, geraniol, nerol, terpineol, dihydromyrcenol, ethyllinalool, farnesol, nerolidol, cis-3-hexenol, menthol, borneol, β-phenylethyl alcohol, benzyl alcohol, phenyl hexanol, 2,2,6-trimethylcyclohexyl-3-hexanol, 1-(2-t-butylcyclohexyloxy)-2-butanol, 4-isopropylcyclohexane methanol, 4-methyl-2-(2-methylpropyl)tetrahydro-2H-pyran-4-ol, 2-methyl-4-(2,2,3-trimethyl-3-cyclopenten-1-yl)-2-butene-1-ol, 2-ethyl-4-(2,2,3-trimethyl-3-cyclopenten-1-yl)-2-buten-1-ol, isocamphylcyclohexanol, and 3,7-dimethyl-7-methoxyoctane-2-ol; phenols such as eugenol, thymol, and vanillin; esters such as linalyl formate, citronellyl formate, geranyl formate, n-hexyl acetate, cis-3-hexenyl acetate, linalyl acetate, citronellyl acetate, geranyl acetate, neryl acetate, terpinyl acetate, nopyl acetate, bornyl acetate, isobronyl acetate, o-t-butylcyclohexyl acetate, p-t-butylcyclohexyl acetate, tricyclodecenyl acetate, benzyl acetate, styralyl acetate, cinnamyl acetate, dimethylbenzylcarbinyl acetate, 3-pentyltetrahydropyran-4-yl acetate, citronellyl propionate, tricyclodecenyl propionate, allylcyclohexyl propionate, ethyl-2-cyclohexyl propionate, benzyl propionate, citronellyl butyrate, dimethylbenzylcarbinyl n-butyrate, tricyclodecenyl isobutyrate, methyl-2-nonenoate, methyl benzoate, benzyl benzoate, methyl cinnamate, methyl salicylate, n-hexyl salicylate, cis-3-hexenyl salicylate, geranyl tiglate, cis-3-hexenyl tiglate, methyl jasmonate, methyldihydro jasmonate, methyl-2,4-dihydroxy-3,6-dimethyl benzoate, ethylmethylphenyl glycidate, methyl anthranilate, and FRUITATE; aldehydes such as n-octanal, n-decanal, n-dodecanal, 2-methylundecanal, 10-undecenal, citronellal, citral, dimethyl tetrahydrobenzaldehyde, 4(3)-(4-hydroxy-4-methylpentyl)-3-cyclohexene-1-carboaldehyde, 2-cyclohexyl propanal, p-t-butyl-α-methylhydrocinnamic aldehyde, p-isopropyl-α-methylhydrocinnamic aldehyde, p-ethyl-α,α-dimethylhydrocinnamic aldehyde, α-amylcinnamic aldehyde, α-hexylcinnamic aldehyde, piperonal, and α-methyl-3,4-methylenedioxyhydrocinnamic aldehyde; ketones such as methylheptenone, 4-methylene-3,5,6,6-tetramethyl-2-heptanone, amylcyclopentanone, 3-methyl-2-(cis-2-pentene-1-yl)-2-cyclopentene-1-on, methylcyclopentenolone, rose ketones, γ-methylionone, α-ionone, carbone, menthone, camphor, nootkatone, benzylacetone, anisylacetone, methyl-β-naphthylketone, 2,5-dimethyl-4-hydroxy-3(2H)-furanone, maltol, 7-acetyl-1,2,3,4,5,6,7,8-octahydro-1,1,6,7-tetramethyl naphthalene, muscone, civetone, cyclopentadecanone, and cyclohexedecenone; acetals and ketals such as acetoaldehyde ethylphenylpropyl acetal, citraldiethyl acetal, phenylacetoaldehyde glycerin acetal, and ethylacetoacetate ethyleneglycol ketals; ethers such as anethole, β-naphthylmethyl ether, β-naphthylethyl ether, limonene oxide, rose oxide, 1,8-cineol, and racemic or photoactive dodecahydro-3a,6,6,9a-tetramethylnaphtho[2,1-b]furane; nitriles such as citronellyl nitrile; lactones such as γ-nonalactone, γ-undecalactone, α-decalactone, γ-jasmolactone, coumarin, cyclopentadecanolide, cyclohexadecanolide, ambrettolide, ethylene brassylate, and 11-oxahexadecanolide; and natural essential oils and natural extracts of orange, lemon, bergamot, mandarin, peppermint, spearmint, lavender, chamomile, rosemary, eucalyptus, sage, basil, rose, geranium, jasmine, ylang-ylang, anise, clove, ginger, nutmeg, cardamom, cedar, Japanese cypress, vetiver, patchouli, and labdanum. One of these fragrance substances may be individually blended, or two or more of them may be blended.

The fragrance composition of the present invention can be used as an aromatic component in various products such as perfumes and cosmetics, health and hygiene materials, sundry goods, beverages, food products, quasi-drugs, and pharmaceuticals, to add aromas as well as to improve the aromas of the products to which the fragrance composition is blended.

The fragrance composition of the present invention can be used as an aromatic component, for example, in fragrance products such as perfumes and colognes; cosmetics for hair, such as shampoos, conditioners, hair tonics, hair creams, mousses, gels, pomades, an sprays; cosmetics for skin, such as lotions, beauty essences, creams, emulsions, packs, foundations, face powders, lipsticks, and makeup cosmetics; products for hygiene, such as dish washing detergents, laundry washing detergents, house detergents, glass cleaners, softeners, bleaching agents, aromatic agents, and insecticides; quasi-drugs, such as toothpastes, mouthwashes, bath additives, antiperspirants, and permanent wave agents; sundry goods, such as paper products; pharmaceuticals; and food products.

A content of the fragrance composition of the present invention in the above products is to be appropriately adjusted according to the intensity of the scent required for each product and the like, and is preferably from 0.001 to 50 mass% and more preferably from 0.01 to 20 mass%.

### [Method for Producing Alicyclic Alcohol and Method for Producing Alicyclic Alcohol Composition]

The alicyclic alcohol of the present invention and the alicyclic alcohol constituting the alicyclic alcohol composition of the present invention may be obtained by any production method.

Among others, the method for producing an alicyclic alcohol of the present invention is preferably a method of hydrogenating an aromatic aldehyde represented by General Formula (5) below to obtain an alicyclic alcohol represented by General Formula (6) below.

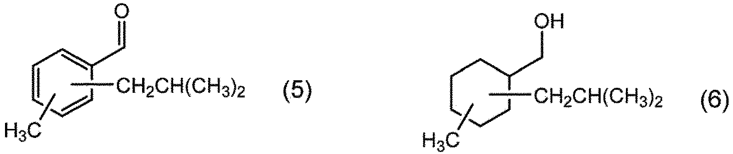

The aromatic aldehyde represented by General Formula (5) is preferably an aromatic aldehyde represented by General Formula (7) below. Therefore, a more preferable method for producing an alicyclic alcohol of the present invention is a method of hydrogenating an aromatic aldehyde represented by General Formula (7) below to obtain an alicyclic alcohol represented by General Formula (1) below.

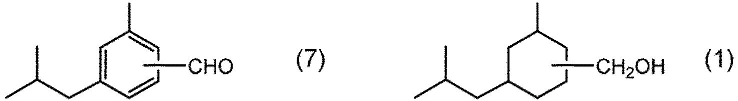

The aromatic aldehyde represented by General Formula (7) includes an aromatic aldehyde represented by General Formula (7-1) below. Furthermore, the aromatic aldehyde represented by formula (7-1) below is an aromatic aldehyde represented by formula (7-2) below or an aromatic aldehyde represented by formula (7-3) below. 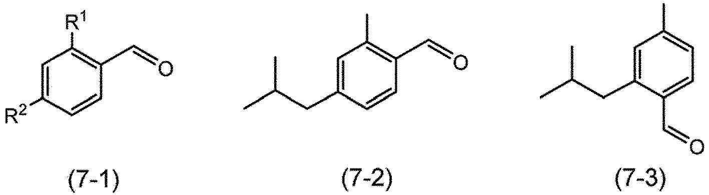 where R¹ is a methyl group and R² is an isobutyl group, or R¹ is an isobutyl group and R² is a methyl group.

According to the present production method, the alicyclic alcohol represented by General Formula (2) can be obtained from the aromatic aldehyde represented by General Formula (7-1). Specifically, the alicyclic alcohol represented by formula (3) can be obtained from the aromatic aldehyde represented by formula (7-2), and the alicyclic alcohol represented by formula (4) can be obtained from the aromatic aldehyde represented by formula (7-3).

The alicyclic alcohol composition containing the alicyclic alcohol represented by formula (3) and the alicyclic alcohol represented by formula (4) of the present invention may be obtained directly by hydrogenating a mixture of the aromatic aldehyde represented by formula (7-2) and the aromatic aldehyde represented by formula (7-3) as raw materials, or may be obtained by obtaining each of the alicyclic alcohols and then mixing them.

The aromatic aldehyde represented by General Formula (5) to be used as a raw material for the hydrogenation reaction may be obtained by any method, but is preferably synthesized from isobutyltoluene by formylation using a super-strong acid as shown in the following formula:

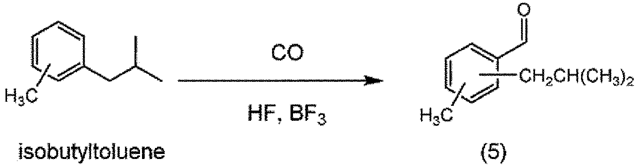

The formylation to obtain the aromatic aldehyde represented by formula (5) to be used in the production method of the present invention is preferably a method in which carbon monoxide is reacted with isobutyltoluene in the presence of a super-strong acid, and is specifically preferably a method in which carbon monoxide is reacted with isobutyltoluene in the presence of a Brønsted acid and a Lewis acid, such as HF/BF₃.

When HF/BF₃ is used as the Brønsted acid and the Lewis acid, the amount of HF to be used is preferably from 5 to 25 mol times the amount of raw material isobutyltoluene, and the amount of BF₃ to be used is preferably from 0.2 to 2.5 mol times the amount of raw material isobutyltoluene.

The reaction temperature is preferably from -30 to 10°C.

The reaction is preferably conducted under pressure, and the reaction pressure is preferably from 1.0 to 3.0 MPaG.

The reaction time is preferably from 0.2 to 5 hours.

A method of adjusting the proportion in the aromatic aldehyde mixture may be to adjust the conditions for the formylation reaction. The ratio in the resulting aromatic aldehyde mixture may be adjusted by distillation. The aromatic aldehyde mixture containing the aromatic aldehyde represented by formula (7-2) and the aromatic aldehyde represented by formula (7-3) in any ratio can be thus obtained.

The aromatic aldehyde or the aromatic aldehyde mixture thus obtained may be used as is in the hydrogenation reaction, but may be further purified to increase the purity of the target aromatic aldehyde. Alternatively, it may be separated by distillation or the like, or mixed with another aromatic aldehyde or aromatic aldehyde mixture to adjust the proportion to a desired proportion.

The reaction conditions of the hydrogenation reaction in the production method of the present invention are not particularly limited, but it is preferred to place the aromatic aldehyde or the aromatic aldehyde mixture described above, a hydrogenation catalyst, and as necessary a solvent and the like in a pressure vessel and to introduce hydrogen.

The hydrogenation catalyst to be used in the present reaction is any typical metal catalyst used in a hydrogenation. Specific examples include ruthenium catalysts, palladium catalysts, platinum catalysts, cobalt catalysts, and nickel catalysts. From the viewpoint of being able to simultaneously hydrogenate the aromatic ring and the aldehyde group, the hydrogenation catalyst is preferably at least one selected from the group consisting of ruthenium catalysts, palladium catalysts, and platinum catalysts and more preferably a ruthenium catalyst.

The support of the metal catalyst includes activated carbon, alumina, and diatomite, and is preferably activated carbon.

In the present reaction, a solvent may be used, and an alicyclic hydrocarbon compound or a saturated aliphatic hydrocarbon compound which is a solvent that does not affect hydrogenation reaction is preferably used. However, a solvent is preferably not substantially used as this eliminates the need for removing the solvent.

The hydrogen to be used in the present reaction need not be a particularly purified hydrogen, and may be an industrial grade hydrogen. The hydrogen pressure during the reaction is preferably from 2.0 to 20.0 MPa, more preferably from 3.0 to 15.0 MPa, and even more preferably from 5.0 to 10.0 MPa.

The reaction temperature is preferably from 80 to 150°C and more preferably from 110 to 140°C. Conducting the reaction in these ranges can suppress side reactions and produce a target product in high yield, which is preferred.

The hydrogenation reaction is preferably conducted to simultaneously hydrogenate the aromatic ring and the aldehyde group and obtain the target alicyclic alcohol or alicyclic alcohol composition in one pot, but the reaction may be divided into two stages to hydrogenate the aromatic ring and the aldehyde group separately.

The alicyclic alcohol or alicyclic alcohol composition thus obtained is preferably purified by distillation. The distillation conditions are to be appropriately adjusted by the pressure and temperature during the distillation.

The alicyclic alcohol represented by General Formula (1), the alicyclic alcohol represented by General Formula (2), the alicyclic alcohol represented by formula (3) and the alicyclic alcohol represented by formula (4) can thus be obtained. Further, the alicyclic alcohol composition containing two or more alicyclic alcohols of the alicyclic alcohols represented by General Formula (1), and the alicyclic alcohol composition containing both the alicyclic alcohol represented by formula (3) and the alicyclic alcohol represented by formula (4) can thus be obtained. These can each be used as fragrance but can also be mixed in any ratio and used. Among others, the alicyclic alcohol composition containing the alicyclic alcohol represented by formula (3) and the alicyclic alcohol represented by formula (4) in the above proportions is preferred.

The obtained alicyclic alcohol represented by General Formula (1) has a floral and fruity scent, and thus is useful as fragrance, and further can emphasize a juicy feeling, a floral feeling, and a fruity feeling in addition to the scent in a compounded fragrance, and thus is also useful as an ingredient for a compounded fragrance (fragrance composition).

### Examples

The present invention will be described specifically based on Examples described below, but the present invention is not limited to these Examples.

### <Component Proportion of Alicyclic Alcohol and Composition Ratio of Alicyclic Alcohol Composition>

Component proportions and composition ratios of products obtained in the Examples were calculated and determined, according to the following equation, from the area of each peak in a chromatograph obtained by a gas chromatography analysis method under the following conditions. The composition ratio ((3)/(4) ratio) of the alicyclic alcohol composition was calculated from a ratio of the proportions of the respective alicyclic alcohols in the alicyclic alcohol composition, the proportions being determined according to the following equation. Component proportion (%) of alicyclic alcohol = peak area of target alicyclic alcohol/total area of all peaks excluding solvent × 100 Proportion (%) of target alicyclic alcohol (alicyclic alcohol represented by Formula (3) or alicyclic alcohol represented by Formula (4)) in alicyclic alcohol composition = peak area of target alicyclic alcohol/total peak area of alicyclic alcohol represented by Formula (3) and alicyclic alcohol represented by Formula (4) × 100

### (Gas Chromatography Analysis)

Instrument: Gas Chromatography Nexis GC-2030 (available from Shimadzu Corporation)
Column: DB-1 (nonpolar, 100% polydimethylsiloxane, 30 m in length, 0.53 mm in inner diameter, and 1.5 µm in film thickness)
Detector: FID (H₂ 30 mL/min, Air 300 mL/min)
Carrier gas: He (constant flow; average linear velocity 38 cm/sec)
Split ratio: 28.1
Injection port temperature: 300°C
Detector temperature: 300°C
Injection volume: 1.0 µL
Oven temperature: The temperature was raised from 50°C to 150°C at 5°C/min, then raised to 280°C at 10°C/min after reaching 150°C, and then maintained for 7 minutes. The temperature was then raised to 300°C at 10°C/min and maintained for 5 minutes.

### <Structure of Compound>

The alicyclic alcohols and compositions obtained in the Examples were subjected to NMR analysis and GC-MS analysis (gas chromatography mass spectrometry) under the following conditions.

### (NMR Analysis Method)

Instrument used: Varian NMR System PS600 600 MHz NMR spectrometer
Resonance frequency: 600 MHz
Measurement temperature: room temperature
Measurement range: -2 ≤ δ ≤ 14
Solvent: CDCl₃
Chemical shift reference material: CHCl₃ in CDCl₃ (δ = 7.26)

### (GC-MS Analysis Method)

### (GC Side)

Instrument used: Gas Chromatography GC2010 Plus (available from Shimadzu Corporation)
Column: DB-1MS (30 m in length, 0.25 mm in inner diameter, and 0.25 µm in film thickness)
Injection volume: 1 µL
Gasification chamber temperature: 300°C
Carrier gas: He
Linear velocity control: 38.0 cm/s
Split ratio: 28.1
Oven temperature: The temperature was raised from 50°C to 150°C at 5°C/min, then raised to 280°C at 10°C/min after reaching 150°C, and then maintained for 7 minutes. The temperature was then raised to 300°C at 10°C/min and maintained for 5 minutes.

### (MS Side)

Instrument used: GCMS-QP2010 Ultra (available from Shimadzu Corporation)
Fragmentation method: EI (Example 2), CI (Example 3)
Ion source temperature: 200°C
Interface temperature: 250°C
Detector voltage: 0.8 kV

### Production Example 1 (Production of Aromatic Aldehyde Mixture)

As the formylation reactor, used was a 500-mL autoclave equipped with a magnet drive-type stirrer and 3 inlet nozzles at the top and 1 outlet nozzle at the bottom, the internal temperature of which autoclave was controllable with a jacket. A refrigerant was allowed to flow through the jacket, and 104.6 g (5.23 mol) of hydrogen fluoride was placed in the autoclave cooled to -25°C. Thereafter, 79.9 g (1.18 mol) of boron trifluoride was added with stirring while the temperature was adjusted not to exceed -25°C. After boron trifluoride was added, the pressure was raised to 2 MPaG with carbon monoxide while the temperature in the autoclave was maintained at -25°C, and 97.8 g (0.66 mol) of m-isobutyltoluene was added thereto.

After stirred for 240 minutes while the temperature of -25°C and the pressure of 2 MPaG were maintained, the reaction mixture in the autoclave was extracted into ice water. The extracted mixture was shaken well, and then the oil layer was separated. The resulting oil layer part was washed with water, and an aromatic aldehyde mixture was obtained.

### Example 1 (Production of Alicyclic Alcohol Composition)

A hydrogenation reaction was conducted using the aromatic aldehyde mixture obtained in Production Example 1.

In a 500-mL autoclave were placed 150.0 g of the aromatic aldehyde mixture and 16.5 g (dry weight of 7.5 g) of a 5 mass% Ru/C catalyst with a water content of 54.7%, and the inside of the reactor was purged with nitrogen and hydrogen. Then, hydrogen was introduced to give a hydrogen pressure of 8.0 MPa, the reaction was conducted with stirring at a reaction temperature of 140°C for six hours (the reaction was started at the start of temperature rise), and an alicyclic alcohol composition was obtained. The end point of the reaction was a point when the consumption of hydrogen ended.

The resulting alicyclic alcohol composition was rectified using a rectifying column with a theoretical plate number of 80 stages (distillation temperature: 132°C and degree of vacuum: 10 torr), and an alicyclic alcohol composition was obtained.

The resulting alicyclic alcohol composition was an alicyclic alcohol composition containing (4-isobutyl-2-methylcyclohexyl)methanol (Formula (3)) and (2-isobutyl-4-methylcyclohexyl)methanol (Formula (4)) in a peak area ratio of 89.1:10.9 in a gas chromatograph. Further, the total purity of (4-isobutyl-2-methylcyclohexyl)methanol (Formula (3)) and (2-isobutyl-4-methylcyclohexyl)methanol (Formula (4)) was 99.9% in terms of a peak area in a gas chromatograph.

The obtained alicyclic alcohol composition had a floral and fruity scent.

### Example 2 (Production of Alicyclic Alcohol (4-isobutyl-2-methylcyclohexyl)methanol (Formula (3)))

The aromatic aldehyde mixture obtained in Production Example 1 was repeatedly rectified using a rectifying column with a theoretical plate number of 80 stages (distillation temperature: 150°C and degree of vacuum: 9 torr), and 4-isobutyl-2-methylbenzaldehyde was obtained. The purity of the obtained 4-isobutyl-2-methylbenzaldehyde was 98.6% in terms of a peak area in a gas chromatograph. It also contained 0.7% of 2-isobutyl-4-methylbenzaldehyde and 0.7% of other compounds.

Obtained was (4-isobutyl-2-methylcyclohexyl)methanol (Formula (3)) in the same manner as in Example 1 using the obtained 2-isobutyl-4-methylbenzaldehyde.

The obtained (4-isobutyl-2-methylcyclohexyl)methanol (Formula (3)) had a purity of 98.6% in terms of a peak area in a gas chromatograph.

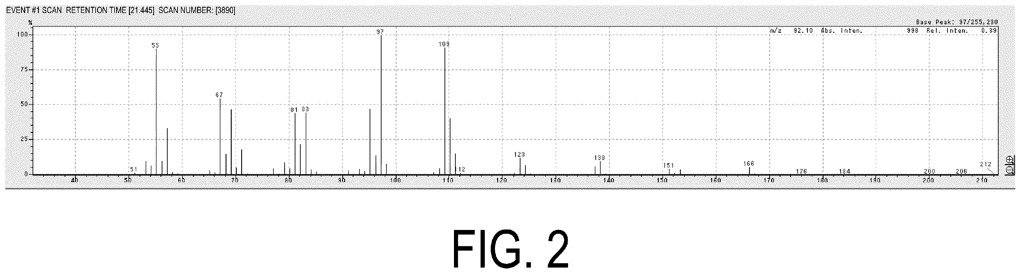

FIGS. 1-1 to 1-5 illustrate NMR analytical results of the alicyclic alcohol (4-isobutyl-2-methylcyclohexyl)methanol (Formula (3)) obtained in Example 2. FIGS. 1-2 to 1-5 are enlarged views of a part of the NMR analytical result illustrated in FIG. 1-1. FIG. 2 illustrates a GC-MS analytical result of the alicyclic alcohol (4-isobutyl-2-methylcyclohexyl)methanol (Formula (3)) obtained in Example 2.

### Example 3 (Production of alicyclic alcohol (2-isobutyl-4-methylcyclohexyl)methanol (Formula (4)))

The aromatic aldehyde mixture obtained in Production Example 1 was repeatedly rectified using a rectifying column with a theoretical plate number of 80 stages (distillation temperature : 150°C and degree of vacuum: 9 torr), and 2-isobutyl-4-methylbenzaldehyde was obtained.

Obtained was (2-isobutyl-4-methylcyclohexyl)methanol (Formula (4)) in the same manner as in Example 1 using the obtained 2-isobutyl-4-methylbenzaldehyde.

The purity of the obtained (2-isobutyl-4-methylcyclohexyl)methanol (Formula (4)) was 92.6% in terms of a peak area in a gas chromatograph.

FIGS. 3-1 to 3-5 illustrate NMR analytical results of the alicyclic alcohol (2-isobutyl-4-methylcyclohexyl)methanol (Formula (4)) obtained in Example 3. FIGS. 3-2 to 3-5 are enlarged views of a part of the NMR analytical result illustrated in FIG. 3-1. FIG. 4 illustrates a GC-MS analytical result of the alicyclic alcohol (2-isobutyl-4-methylcyclohexyl)methanol (Formula (4)) obtained in Example 3.

### <Evaluation of Aroma of Alicyclic Alcohol Composition and Alicyclic Alcohol>

The alicyclic alcohol compositions and the respective alicyclic alcohols obtained in Examples 1 to 3 were evaluated for aromas. In the evaluation of the aroma, those having an excellent aroma were rated as B, and those having an extremely excellent aroma were rated as A. The details are shown in Table 1.

### [Table 1]

**Table 1**

| | Example 1 | Example 2 | Example 3 |
|---|---|---|---|
| (4-Isobutyl-2-methyl) cyclohexyl)methanol (3) | 89.1 | 98.6 | 6.3 |
| (2-Isobutyl-4-methyl cyclohexyl)methanol (4) | 10.9 | 0.7 | 92.6 |
| (3)/(4) Ratio | 89.1/10.9 | 99.3/0.7 | 6.4/93.6 |
| Other compounds | 0.1 | 0.7 | 1.1 |
| Evaluation of aromas | A | B | B |
| | Floral and fruity | Floral and fruity | Floral and fruity |
| Comparison with Example 1 | - | Having rhubarb-like bitterness and chemical green aroma | Having a low-intensity scent |

| | | | |
|---|---|---|---|
| *) The number in the table represents the content (purity) of each compound in terms of a peak area ratio (%) in a gas chromatograph. | | | |

### Example 4 and Comparative Examples 1 and 2 (sugar blossom-like fragrance compositions)

A fragrance composition was obtained by adding 5 parts by mass of the alicyclic alcohol composition obtained in Example 1 to the compounded fragrance bases (sugar blossom-like) listed in Table 2, and was evaluated for the aroma. Also, a fragrance composition obtained by adding 5 parts by mass of dipropylene glycol instead of the alicyclic alcohol composition was used in Comparative Example 1, which was then evaluated for the aroma in the same manner as for the fragrance composition of Example 4, and comparison with Example 4 was made. A fragrance composition obtained by adding 5 parts by mass of FLOROPAL (2,4,6-trimethyl-4-phenyl-1,3-dioxane, fresh herbal, green, floral fragrance) instead of the alicyclic alcohol composition was used in Comparative Example 2, and evaluated for the aroma in the same manner as for the fragrance composition of Example 4, and comparison with Example 4 was made.

### [Table 2]

**Table 2**

| Blended components | Example 4 | Comparative Example 1 | Comparative Example 2 |
|---|---|---|---|
| | (parts by mass) | (parts by mass) | (parts by mass) |
| Alicyclic alcohol composition (Example 1) | 5.00 | - | - |
| FLOROPAL | - | - | 5.00 |
| Decylaldehyde | 1.00 | 1.00 | 1.00 |
| Dodecanal | 0.04 | 0.04 | 0.04 |
| Octylaldehyde | 0.16 | 0.16 | 0.16 |
| p-Propenylanisole | 0.70 | 0.70 | 0.70 |
| L-Carvone | 0.04 | 0.04 | 0.04 |
| Cedarwood atlas essential oil | 0.40 | 0.40 | 0.40 |
| cis-3-Hexenyl acetate | 0.10 | 0.10 | 0.10 |
| Neral | 5.00 | 5.00 | 5.00 |
| Citronellol | 2.00 | 2.00 | 2.00 |
| Citronellyl acetate | 1.50 | 1.50 | 1.50 |
| Dihydromyrcenol | 2.40 | 2.40 | 2.40 |
| Dimethyl anthranilate | 0.05 | 0.05 | 0.05 |
| Dimethyloctenone | 0.40 | 0.40 | 0.40 |
| Dipropylene glycol | 12.71 | 17.71 | 12.71 |
| Ethyl vanillin | 0.10 | 0.10 | 0.10 |
| Ethylene brassylate | 2.50 | 2.50 | 2.50 |
| Geranyl acetate | 2.00 | 2.00 | 2.00 |
| Hedione | 10.00 | 10.00 | 10.00 |
| Piperonal | 1.00 | 1.00 | 1.00 |
| Lime terpene | 10.00 | 10.00 | 10.00 |
| Linalool | 8.50 | 8.50 | 8.50 |
| Linalyl acetate | 10.00 | 10.00 | 10.00 |
| tert-Butylbenzylpropionaldehyde | 16.00 | 16.00 | 16.00 |
| Methyl chavicol | 0.40 | 0.40 | 0.40 |
| Nerol | 1.80 | 1.80 | 1.80 |
| γ-Nonalactone | 0.40 | 0.40 | 0.40 |
| 1-Phenyl ethyl acetate | 2.80 | 2.80 | 2.80 |
| α-Terpineol | 3.00 | 3.00 | 3.00 |
| Total | 100.00 | 100.00 | 100.00 |

As compared with the fragrance compositions of Comparative Examples 1 and 2, the fragrance composition of Example 4 was more emphasized in sweet juicy feeling, fruity feeling of the top note, and floral feeling of the middle note.

### Example 5 and Comparative Examples 3 and 4 (Rhubarb-like Fragrance Compositions)

A fragrance composition was obtained by adding 3 parts by mass of the alicyclic alcohol composition obtained in Example 1 to the compounded fragrance bases (rhubarb-like) listed in Table 3, and was evaluated for the aroma. A fragrance composition obtained by adding 3 parts by mass of dipropylene glycol instead of the alicyclic alcohol composition was used in Comparative Example 3, and evaluated for the aroma in the same manner as for the fragrance composition of Example 5, and comparison with Example 5 was made. A fragrance composition obtained by adding 3 parts by mass of RHUBOFIX (3,4,4a,5,8,8a-hexahydro-3',7-dimethylspiro[1,4-methanonaphthalene-2(1H),2'-oxirane], pepper oil-like fresh and complex woody, spicy, floral, fruity fragrance) instead of the alicyclic alcohol composition was used in Comparative Example 4, and evaluated for the aroma in the same manner as for the fragrance composition of Example 5, and comparison with Example 5 was made.

### [Table 3]

**Table 3**

| Blended components | Example 5 | Comparative Example 3 | Comparative Example 4 |
|---|---|---|---|
| | (parts by mass) | (parts by mass) | (parts by mass) |
| Alicyclic alcohol composition (Example 1) | 3.00 | - | - |
| RHUBOFIX | - | - | 3.00 |
| Allyl cyclohexanepropionate | 0.08 | 0.08 | 0.08 |
| Allyl heptanoate | 0.40 | 0.40 | 0.40 |
| Amber One | 3.50 | 3.50 | 3.50 |
| Ambroxan | 0.50 | 0.50 | 0.50 |
| 3 -(4-tert-Butylphenyl)propanal | 1.50 | 1.50 | 1.50 |
| cis-3-Hexenol | 0.10 | 0.10 | 0.10 |
| Citronellyl acetate | 0.40 | 0.40 | 0.40 |
| Tricyclo[5.2.1.02,6]dec-4-en-3-yl=propionate | 0.80 | 0.80 | 0.80 |
| 1,1-Dimethyl-2-phenylethyl butyrate | 0.07 | 0.07 | 0.07 |
| Damascenone | 0.06 | 0.06 | 0.06 |
| γ-Decalactone | 0.80 | 0.80 | 0.80 |
| Dipropylene glycol | 22.78 | 25.78 | 22.78 |
| Ethyllinalool | 3.50 | 3.50 | 3.50 |
| Ethylene brassylate | 7.00 | 7.00 | 7.00 |
| Methyl 3,6-dimethyl-2,4-dihydroxybenzoate | 0.06 | 0.06 | 0.06 |
| Floral ozone | 0.12 | 0.12 | 0.12 |
| Hedione | 37.00 | 37.00 | 37.00 |
| Helional | 2.50 | 2.50 | 2.50 |
| Helvetolide | 2.50 | 2.50 | 2.50 |
| Herbanate | 3.50 | 3.50 | 3.50 |
| Hexyl acetate | 0.05 | 0.05 | 0.05 |
| Ligustral | 0.07 | 0.07 | 0.07 |
| Para-tert-butyl-α-methylhydrocinnamic aldehyde | 8.50 | 8.50 | 8.50 |
| 2, 6-Dimethyl-5-heptenal | 0.01 | 0.01 | 0.01 |
| 5-(2,2,3-Trimethyl-3-cyclopenten-1-yl)-3-methylpentan-2-ol | 1.00 | 1.00 | 1.00 |
| 2,2,6-Trimethyl-α-propylcyclohexane-1-propanol | 0.20 | 0.20 | 0.20 |
| Total | 100.00 | 100.00 | 100.00 |

As compared with the fragrance compositions of Comparative Examples 3 and 4, the fragrance composition of Example 5 was emphasized in fruity feeling and floral feeling, and, further, could be improved in diffusibility.

From the results of Examples 1 to 3, it can be seen that the alicyclic alcohol and the alicyclic alcohol composition of the present invention have a floral and fruity scent and are therefore useful as fragrance.

In addition, as presented in Examples 4 and 5, the alicyclic alcohol composition of the present invention can emphasize the juicy feeling of the fragrance composition, emphasize the fruity feeling and floral feeling thereof, and impart the diffusibility thereto, as compared with FLOROPAL having another floral aroma and RHUBOFIX having a floral and fruity scent, and thus it can be seen that the alicyclic alcohol composition is also useful as an ingredient for a compounded fragrance.

## Claims

1. An alicyclic alcohol represented by General Formula (1) below:

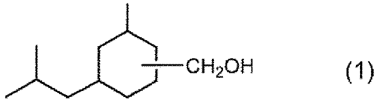

2. The alicyclic alcohol according to claim 1, which is represented by General Formula (2) below: 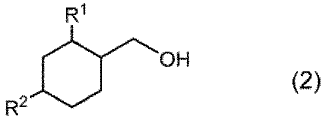 wherein R¹ is a methyl group and R² is an isobutyl group, or R¹ is an isobutyl group and R² is a methyl group.

3. The alicyclic alcohol according to claim 1 or 2, which is represented by Formula (3) below:

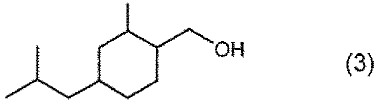

4. The alicyclic alcohol according to claim 1 or 2, which is represented by Formula (4) below:

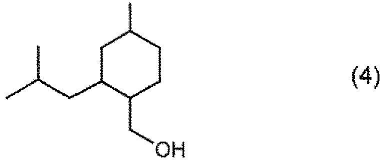

5. An alicyclic alcohol composition comprising an alicyclic alcohol represented by Formula (3) below and an alicyclic alcohol represented by Formula (4) below:

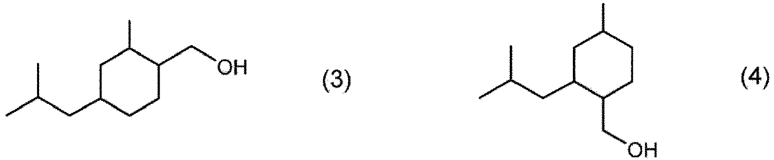

6. The alicyclic alcohol composition according to claim 5, wherein [(3)/(4)], a ratio of the alicyclic alcohol represented by Formula (3) above to the alicyclic alcohol represented by Formula (4) above in terms of a peak area ratio in a gas chromatograph, is from 7/93 to 98/2.

7. A fragrance composition comprising the alicyclic alcohol described in any one of claims 1 to 4 or the alicyclic alcohol composition described in claim 5 or 6.

8. A method for producing an alicyclic alcohol, the method comprising obtaining an alicyclic alcohol represented by General Formula (6) below by hydrogenating an aromatic aldehyde represented by General Formula (5) below:

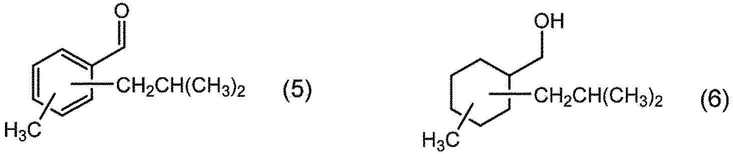

9. The method for producing an alicyclic alcohol, according to claim 8, the method comprising obtaining an alicyclic alcohol represented by General Formula (1) below by hydrogenating an aromatic aldehyde represented by General Formula (7) below:
